(19) Europäisches Patentamt

European Patent Office

Office européen des brevets

(11) **EP 3 897 485 B1**

(12) **EUROPEAN PATENT SPECIFICATION**

(45) Date of publication and mention
of the grant of the patent:
**20.08.2025 Bulletin 2025/34**

(21) Application number: **19832989.8**

(22) Date of filing: **12.12.2019**

(51) International Patent Classification (IPC):
*A61F 7/02* ^(2006.01)      *A61F 7/08* ^(2006.01)

(52) Cooperative Patent Classification (CPC):
**A61F 7/02;** A61F 7/08; A61F 2007/0098;
A61F 2007/0206; A61F 2007/023; A61F 2007/0233;
A61F 2007/0239; A61F 2007/0246;
A61F 2007/0268

(86) International application number:
**PCT/GB2019/053526**

(87) International publication number:
**WO 2020/128436 (25.06.2020 Gazette 2020/26)**

(54) **THERMAL PADS**

WÄRMEKISSEN

TAMPONS THERMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2018 GB 201821090
04.06.2019 GB 201907910
13.09.2019 GB 201913250**

(43) Date of publication of application:
**27.10.2021 Bulletin 2021/43**

(73) Proprietor: **Creation XXI S.A.S
33110 Le Bouscat (FR)**

(72) Inventors:
• **SIVY, Yann
64500 Ciboure (FR)**
• **BLOEDT, Laurent
33110 Le Bouscat (FR)**

(74) Representative: **Vienne, Aymeric Charles Emile et al
Mathys & Squire
The Shard
32 London Bridge Street
London SE1 9SG (GB)**

(56) References cited:
EP-A1- 1 782 778       EP-A1- 1 782 778
EP-A1- 2 881 092       EP-A1- 2 881 092
WO-A2-2014/066842       WO-A2-2014/066842
US-A1- 2017 332 796       US-A1- 2017 332 796
US-A1- 2018 042 762       US-A1- 2018 042 762
US-A1- 2018 049 914

## Description

### Field of Invention

The invention relates but is not limited to a pad for transferring thermal energy to and/or from a body part of a human or an animal. The invention also relates but is not limited to a method of manufacturing the pad.

### Background

Remedies for inflammatory problems (such as tendonitis or post-operation inflammation) and/or muscle problems (such as hematomas or torn muscles) and/or for recovery, well-being and/or for post medical treatment (e.g. against symptoms, side effects, sequela) may include application of heat and/or cold to a body part where the problems are located.

The current devices for applying heat and/or cold are cumbersome and/or not convenient.

EP1,782,778 discloses a heating pad and method of using the same.

US2018/042762 discloses a thermal system.

US2017/332796 discloses back support devices with integrated thermal therapy and methods of using the same.

EP2,881,092 discloses a thermotherapeutic pad and method of manufacturing a thermotherapeutic pad.

WO2014/066842 discloses a cooling medical pad.

### Summary

Aspects and embodiments of the invention are set out in the appended claims. These and other aspects and embodiments of the invention are also described herein.

Methods of treatment are only disclosed as examples of how to use the invention and are not claimed.

### Brief description of Drawings

Aspects and embodiments of the disclosure will now be described, by way of example, with reference to the accompanying drawings in which:

Figure 1 schematically illustrates a cross section of an example device according to the disclosure, forming a loop;
Figure 2 schematically illustrates an elevation view of a first example embodiment of the device of Figure 1 according to the disclosure, in a flat configuration, with a first length and a first width;
Figure 3A schematically illustrates an elevation view of the example device of Figure 2, with a second length and a second width;

Figure 3B schematically illustrates an elevation view of the example device of Figure 2, with a third length and a third width;
Figure 3C schematically illustrates an elevation view of an example device attached to a belt using pins and holes;
Figure 3D schematically illustrates an elevation view of an example device attached to a belt using slots;
Figure 3E schematically illustrates an elevation view of two example devices attached to a belt using slots;
Figure 3F schematically illustrates an elevation view of an example device attached to a belt using slots, on different sides of the device compared to Figure 3D;
Figure 4 schematically illustrates, in an exploded elevation view, the example device of Figure 2, from an outer view into receptacles containing a thermal material;
Figure 5A schematically illustrates an elevation view of a band of a second example embodiment of the device of Figure 1 according to the disclosure, in a flat configuration, from an outer view into receptacles;
Figure 5B schematically illustrates the band of Figure 5A, from a view to pads configured to enable transfer of thermal energy to and/or from a body part of a human or an animal;
Figure 6A schematically illustrates a cross section of a detail of an example receptacle of a device according to the disclosure;
Figure 6B schematically illustrates a cross section of a detail of an example pad of a device according to the disclosure;
Figure 6C schematically illustrates a cross section of a detail of another example pad of a device according to the disclosure;
Figure 6D schematically illustrates an elevated view of a band comprising a plurality of pads of Figure 6C;
Figure 6E schematically illustrates a cross section of a detail of another example pad of a device according to the disclosure;
Figure 7 schematically illustrates a cross section of a detail of another example receptacle of a device according to the disclosure;
Figure 8 schematically illustrates an example method of manufacturing a device according to any aspects of the disclosure;
Figure 9A, 9B, 9C schematically illustrate an example method of manufacturing another device according to any aspects of the disclosure;
Figure 10 schematically illustrates an elevation view of an example device according to the disclosure, comprising one or more thermometer for sending data to a processing device;
Figure 11 schematically illustrates a cross section of an example pad according to the disclosure;
Figure 12 schematically illustrates a cross section of an example pad according to the disclosure, comprising a plurality of curvatures on one face;

Figures 13A to 13C schematically illustrate cross sections of an example pad according to the disclosure attached to a fitting;

Figure 14A schematically illustrates a cross section of a first example pad according to the disclosure, comprising a suction cup;

Figure 14B schematically illustrates a cross section of a second example pad according to the disclosure, comprising a suction cup;

Figure 14C schematically illustrates a cross section of a third example pad according to the disclosure, comprising a suction cup;

Figure 14D schematically illustrates a cross section of a fourth example pad according to the disclosure, comprising a suction cup;

Figure 15 schematically illustrates an example method of manufacturing a pad according to any aspects of the disclosure;

Figures 16A, 16B, 16C, 16D, 16E and 16F schematically illustrate a first example device comprising a fitting for at least one pad according to the disclosure;

Figure 17 schematically illustrates a second example device comprising a fitting for at least one pad according to the disclosure; and

Figures 18A to 18F schematically illustrate several non-limiting examples of shapes and/or locations of the pad on the body part.

In the drawings, similar elements bear identical numerical references.

**Specific Description of Example Embodiments**

Overview

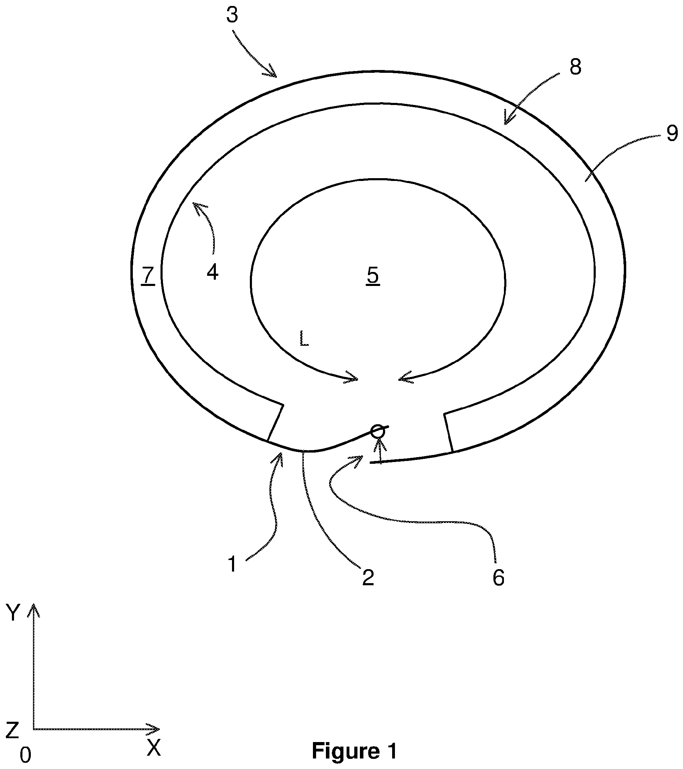

Figure 1 schematically illustrates an example device 1 according to the disclosure. The device 1 comprises a band 2 made of a polymer material. The band 2 extends along a length L in a plane (YOX) of Figure 1, and has a width W in a direction perpendicular to the plane (YOX), e.g. parallel to axis (OZ) of Figure 1. The band 2 has two mains faces, e.g. the faces 3 and 4 in the example of Figure 1. One face, e.g. face 4 in the example of Figure 1, may correspond to an inner face of a loop or a C-shape which may be formed by the band 2 in the plane (YOX). The inner face 4 of the loop or the C-shape may define a receiving space 5 for receiving a body part of a human or an animal. The body part may be at least a part of an arm (such as a wrist, a neck, an elbow or a biceps) or at least a part of a leg (such as a thigh, a knee, an Achilles tendon or an ankle), or any other part of the body of the human or the animal.

The device 1 may also comprise complementary fasteners 6 configured to provide adjustment and/or closing of the loop on the body part of the human or the animal. Alternatively or additionally, in some embodiments, as disclosed in more detail below, the fasteners may be configured to enable attachment of the band to one or more belts configured to provide adjustment and/or closing of the device on the body part of the human or the animal.

The device 1 also comprises one or more pads 7 (only one pad 7 is represented on the example of Figure 1, but the device 1 may comprise more than one pad 7, as will be apparent from the description below).

The one or more pads may be configured to be in contact with the body part of the human or the animal when the body part of the human or the animal is received in the receiving space defined by the loop or the C-shape.

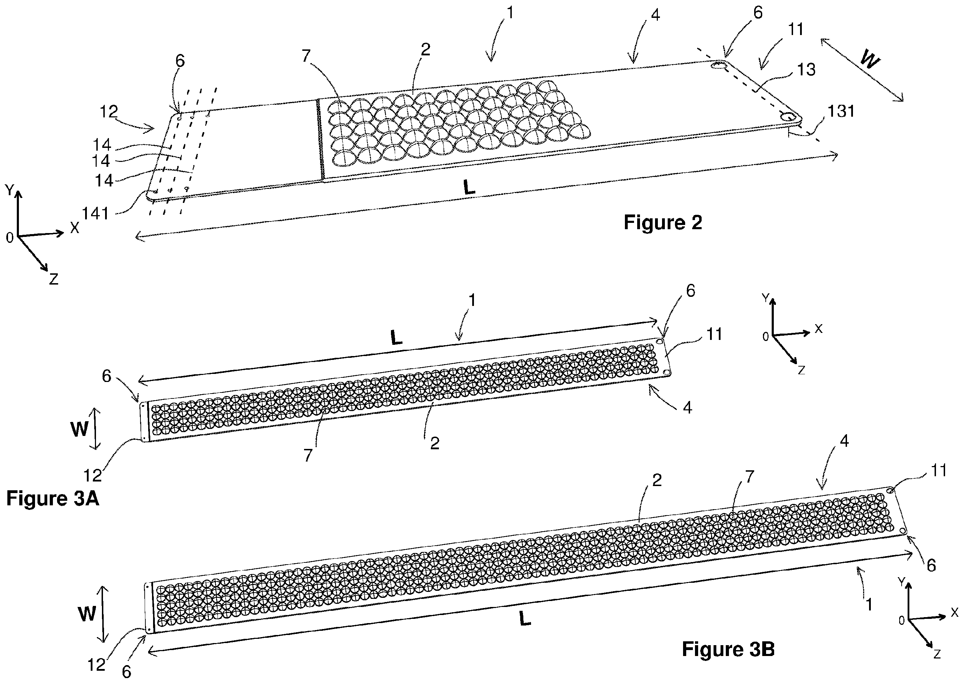

In Figure 1, the one or more thermal pads 7 extend on a surface of the band 2 corresponding substantially to the inner face of the loop or the C-shape defining the receiving space 5. It should be understood that in some examples the extension of the one or more pads 7 may be less than substantially the inner face of the loop or the C-shape defining the receiving space 5. In other words, the one or more thermal pads 7 may extend on a surface of the band 2 which may be for example about 50% (or less) of the band 2 (as shown e.g. in Figure 2), although in some examples the one or more thermal pads 7 may extend on a surface of the band 2 which may be for example about at least 50% (such as 75% or more) of the band 2 (as shown e.g. in Figures 3A and 3B), depending on the part of the body to which the device 1 is adapted. In both cases, the band 2 may extend and/or the complementary fasteners 6 may be located on either side of the one or more pads 7.

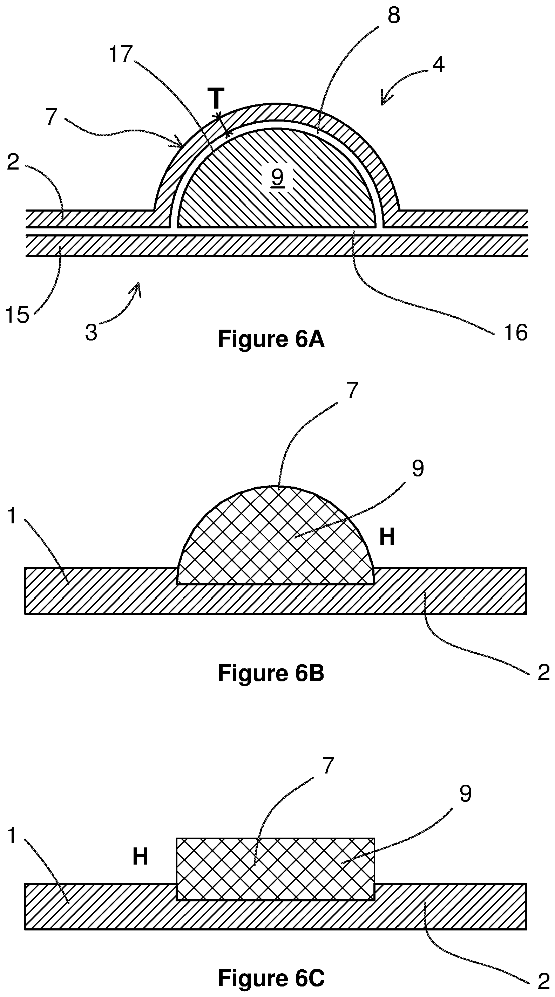

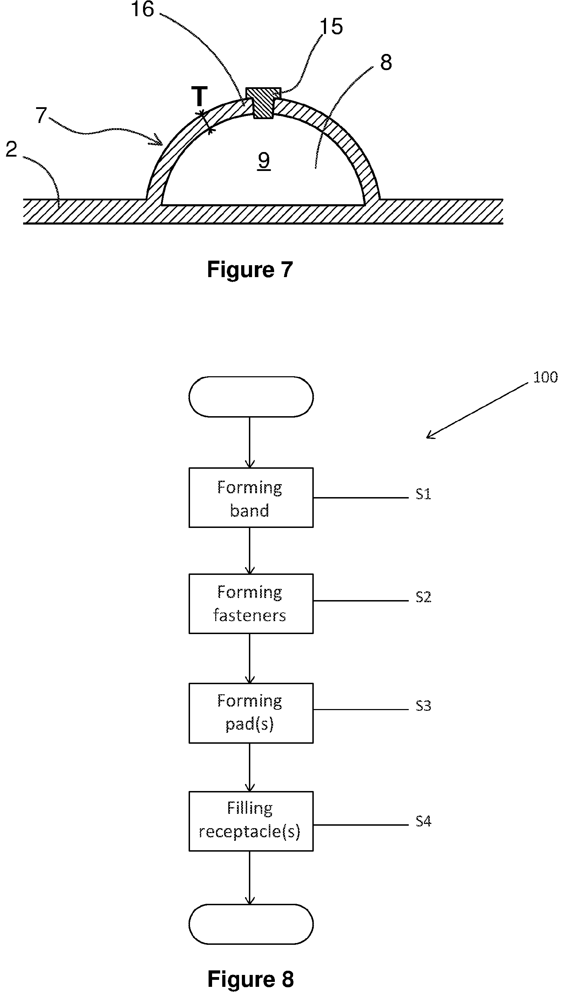

As illustrated in Figures 6A and 7, the pad 7 comprises a receptacle 8 at least substantially made integral with the polymer material of the band 2, and a thermal material 9 located in the receptacle 8. The thermal material 9 is configured to enable transfer of thermal energy to the body part of the human or the animal (e.g. for applying heat to the body part) and/or transfer of thermal energy from the body part of the human or the animal (e.g. for applying cold to the body part) through the receptacle 8, when the body part of the human or the animal is received in the receiving space 5 defined by the loop or the C-shape.

As illustrated in Figures 6A and 7, each receptacle 8 may have a thickness T of polymer material, the thickness T being such that $0.4mm \leq T \leq 15mm$, as non-limiting examples.

In the example of Figures 6A and 7, the thermal material may be a liquid, a solid and/or a gel, such as an aqueous gel and/or a viscous gel, and/or a powder on its own or diluted.

As illustrated in Figure 6B, the thermal pad 7 may be made of a material comprising a mix of a polymer material (e.g. the same polymer material as the polymer material of the band 2 - but it could be a different polymer material) and a thermal material 9. The pad 7, comprising the thermal material 9, is configured to enable transfer of thermal energy to the body part of the human or the animal (e.g. for applying heat to the body part) and/or transfer of thermal energy from the body part of the human or the animal (e.g. for applying cold to the body part) through the pad 7, when the body part of the human or the animal is received in the receiving space defined by the loop or the C-shape.

As illustrated in Figure 6C, the pad 7 may have a flat surface, e.g. may have a shape of a cylinder, e.g. a cylinder of revolution, instead of a spherical dome as illustrated in Figure 6B. However, it should be understood that other shapes are envisaged, such as shapes having a rectangular or square cross-section as non-limiting examples.

In the example of Figures 6B and 6C, after manufacturing, the pad 7 is a solid (i.e. the mix of polymer material and the thermal material is solid).

In the examples of Figures 6B and 6C, the pad 7 is embedded within the polymer material of the band 2.

The height H above which the pad extends above the band 2 is predetermined at manufacturing, depending on desired applications of the device 1. A higher height H enhances thermal energy transfers to/-from the pad 7, and a lower height H reduces thermal energy transfers to/from the pad 7.

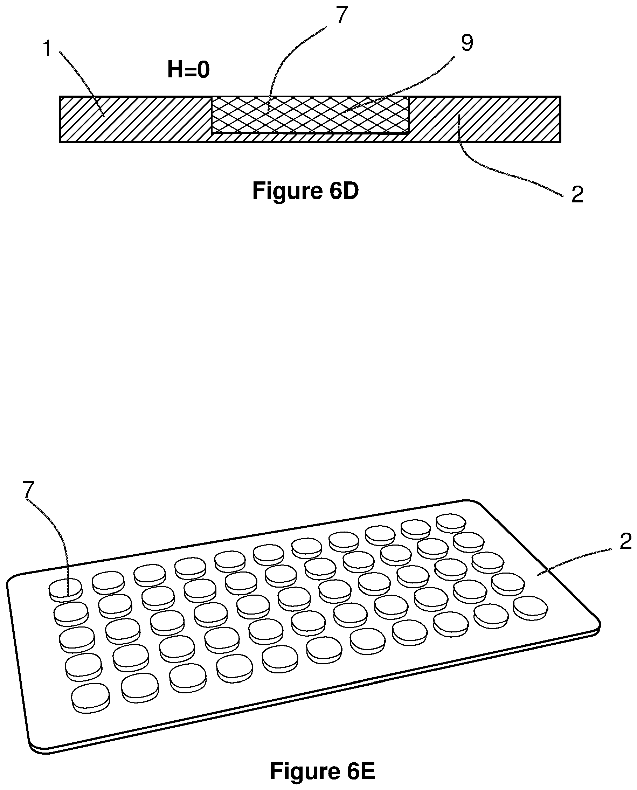

As illustrated in Figure 6E, H may be equal to 0.

The thermal material 9 is configured to be preheated (e.g. the device 1 may be placed in a microwave oven or in an electrical oven or in a hot water bath as non-limiting examples) and/or precooled (e.g. the device 1 may be placed in a refrigerator or a freezer as non-limiting examples), prior to the device 1 being placed on the body part of the human or the animal. The device 1 may be used when necessary, e.g. when heat and/or cold needs to be applied to the body part.

In some examples, each pad 7 may comprise a convex curvature towards the inner face of the loop or the C-shape defining the receiving space 5 (see for example Figures 6A and 6B). The convex curvature enables the band 2 to form the loop or the C-shape and enhances the transfer of thermal energy to and/or from the body part of the human or the animal through the receptacle, when the body part of the human or the animal is received in the receiving space 5 defined by the loop or the C-shape. It should be understood that other shapes may be envisaged for the one or more pads 7 (see for example Figure 6C).

In some examples and as described in greater detail below, the complementary fasteners 6 may comprise a plurality of complementary systems located at a plurality of positions on the band 2, so as to provide the adjustment of the loop or the C-shape to the body part of the human or the animal.

Alternatively or additionally, the complementary fasteners 6 may comprise at least one of one or more headed pins, each forming a button, cooperating with one or more holes, and/or one or more hooks cooperating with one or more slots or rings, so as to provide the closing of the loop or the C-shape on the body part of the human or the animal.

The device 1 may enable applying heat to the body part (resulting in e.g. vasodilatation) and/or applying cold to the body part (resulting in e.g. vasoconstriction). The device 1 may enhance recovery from exercise and/or from inflammatory problems (such as tendonitis or post-operation inflammation) and/or from muscle problems (such as hematomas or torn muscle).

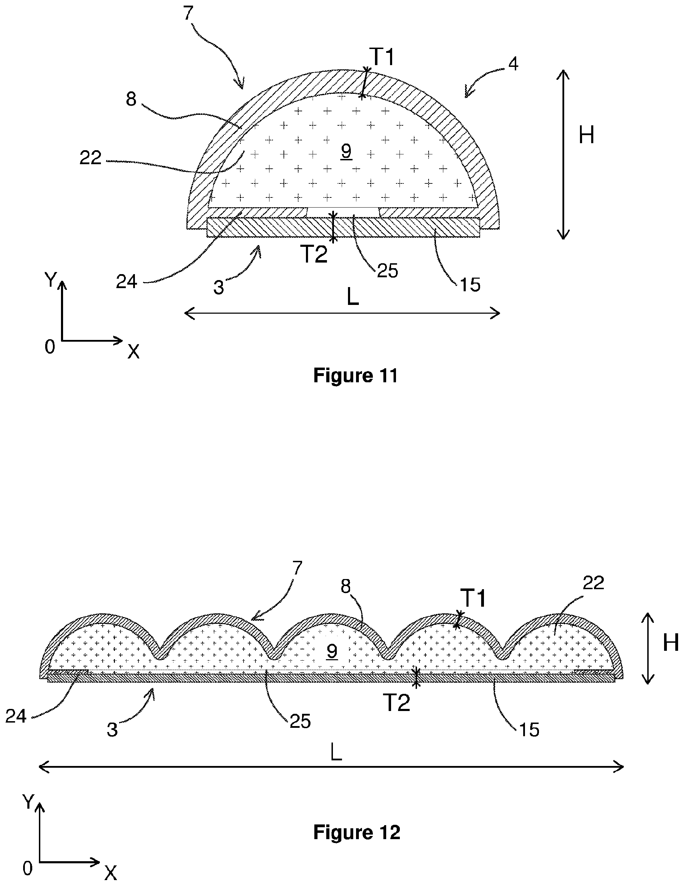

In the examples of Figures 11 and 12, the thermal pad 7 comprises a container 22. The container 22 of the thermal pad 7 comprises a first part 8 (e.g. the receptacle 8 according to any aspects of the disclosure) configured to form a first face 4 of the pad 7, and a second part 15 (e.g. a lid 15 described in greater detail below according to any aspects of the disclosure) configured to form a second face 3 of the pad 7.

It should be understood that the pad 7 of Figures 11 and 12 is configured to be in contact with the body part of the human or the animal on either or both of the first face 4 and the second face 3.

In some examples, the first part 8 may be made of a first polymer material. The first polymer material may comprise at least one of: a silicone, a polyurethane, a thermoplastic elastomer and/or an elastomer.

Alternatively or additionally, the second part 15 may be made of a material comprising a mix of a second polymer material, and a first thermal material. In some examples, the first thermal material may be configured to enable transfer of thermal energy to and/or from the body part of the human or the animal through the second part, when the second face 3 is in contact with the body part of the human or the animal. In some examples, the second polymer material may comprise at least one of: a silicone, a polyurethane, a thermoplastic elastomer and/or an elastomer. In some examples, the material of the second part 15 may be the same material as already described with reference to the pad 7 of Figures 6B and 6C.

Referring back to Figures 11 and 12, a second thermal material 9 may be located in the container 22. As already described above, the second thermal material 9 may be configured to enable transfer of thermal energy to and/or from the body part of the human or the animal through the container 22, when the pad 7 is in contact with the body part of the human or the animal.

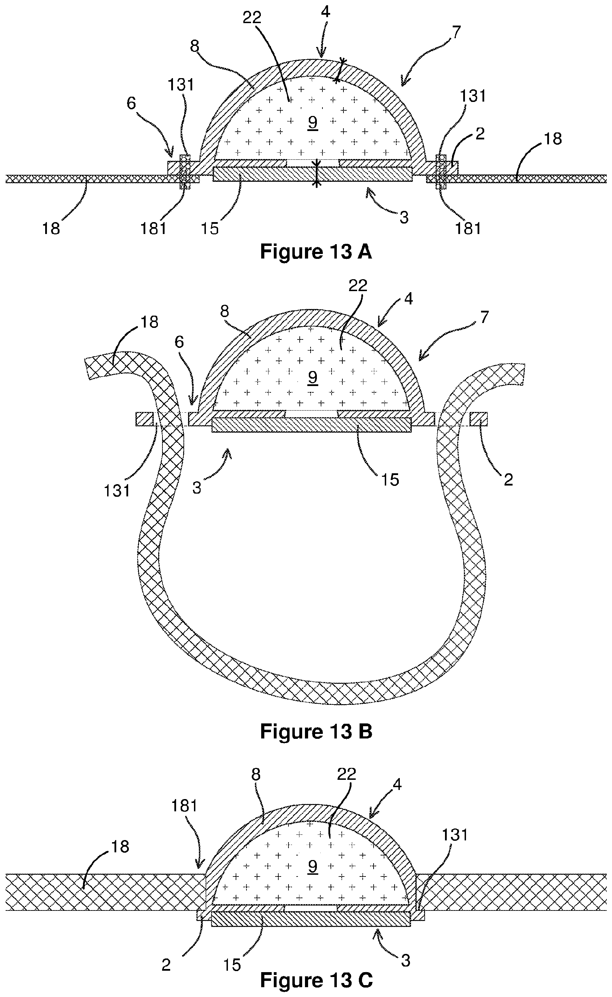

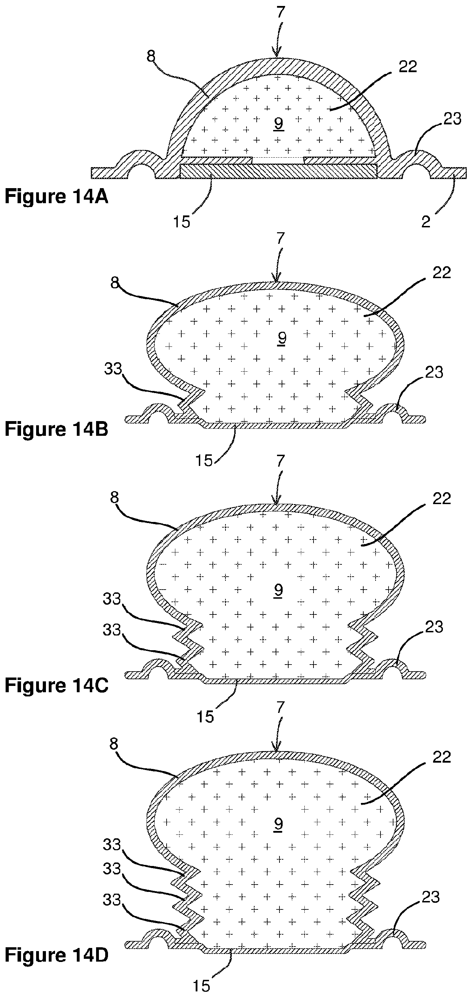

It should be understood that the pad 7 of Figures 11 and 12 may be used on its own (e.g. a user may manually hold the pad 7 in contact with the body part of the human or the animal). Alternatively or additionally, the pad 7 may be held in contact with the body part of the human or the animal using at least one fitting (e.g. one or more belts and/or a jacket described in greater detail later, with reference to Figures 13A to 13C) and/or suction cups (described in greater detail later, with reference to Figures 14A to 14D), as non-limiting examples.

As already stated the body part of the human or the animal may be at least one of:

at least a part of an arm, such as a wrist, a neck, an elbow or a biceps; and/or

at least a part of a leg, such as a thigh, a knee, an Achilles tendon or an ankle, and/or any other part of the body of the human or the animal, such as skin, in case of a skin trauma and/or skin aggression or skin medical operation, as non-limiting examples.

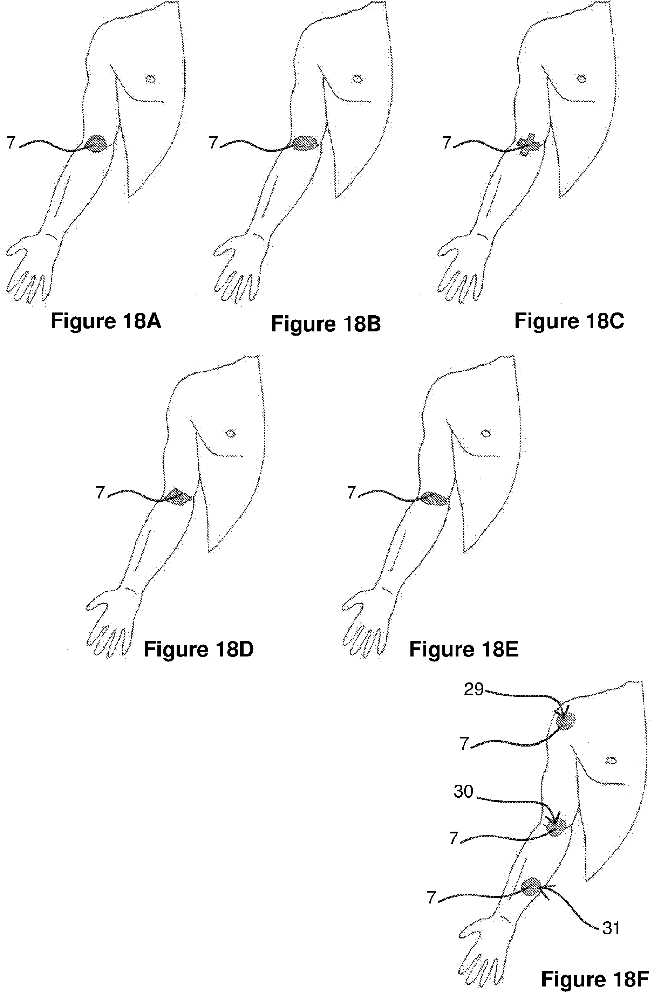

The pad 7 may be applied before and/or after a surgical operation on the skin, for example a vaccination (see e.g. location 29 in Figure 18F) and/or a blood test (see e.g. location 30 in Figure 18F), as non-limiting examples. Alternatively or additionally, the pad 7 may be applied on any aggression of the skin, such as a burn and/or an insect bite (see e.g. location 31 in Figure 18F) as non-limiting examples. In the invention the pad may be applied to the face and forms a mask (in some examples the mask may further be provided with a cream, for further application of the cream to the face). Alternatively or additionally, the pad 7 may be used similarly as a hot water bottle.

In any aspect of the disclosure, the one or more materials forming the container enable transfer of thermal energy to and/or from the body part of the human or the animal (e.g. in the example of Figures 11 and 12, mainly through the second part 15, but also through the first part 8).

In the invention the container further contains one or more further (e.g. medically active) products. The one or more materials forming the container comprising a silicone enable diffusion of the one or more further products located in the container. As non-limiting examples, the one or more further products may comprise at least one of:

one or more scented materials, to provide e.g. an appeasing scent through the container;
a cream;
a serum;
one or more essential oils;
vinegar;
honey;
starch;
one or more products for dilatation of the skin or the pores of the skin; and/or
one or more products for cleaning and/or sanitizing the skin.

It should be understood that the one or more materials forming the container enable preservation of the heat and/or cold which is destined to be transferred through the container (e.g. in the example of Figures 11 and 12, mainly through the second part 15) and/or enable diffusion of a further product contained in the container.

Efficient application of heat and/or of cold and/or of a further product may be therefore enhanced by the pad.

Detailed Description of Example Embodiments

As illustrated in Figure 2, the band 2 comprises a first extremity 11 and a second extremity 12. The complementary fasteners 6 may comprise a line 13 comprising two headed pins 131 located at the first extremity 11 of the band 2, and the plurality of complementary systems may comprise one or more lines 14 comprising two corresponding holes 141 located at the second extremity 12 of the band 2. The line 13 and/or the one or more lines 14 may be substantially perpendicular to the plane (YOX) of Figure 2, e.g. parallel to axis (OZ) illustrated in Figure 2. In some examples, the adjustment of the loop or the C-shape to the body part of the human or the animal may be provided by placing the two headed pins 131 in a line 14 of holes 141 corresponding to a girth of the body part on which the device 1 is placed.

Alternatively or additionally, and as illustrated in Figures 3A and 3B, the band 2 may have a particular length L and a particular width W to correspond to a particular body part.

In some examples, the length L is such that 5cm≤L≤150cm, as non-limiting examples; and/or the width W is such that 3cm≤W≤150cm, as non-limiting examples.

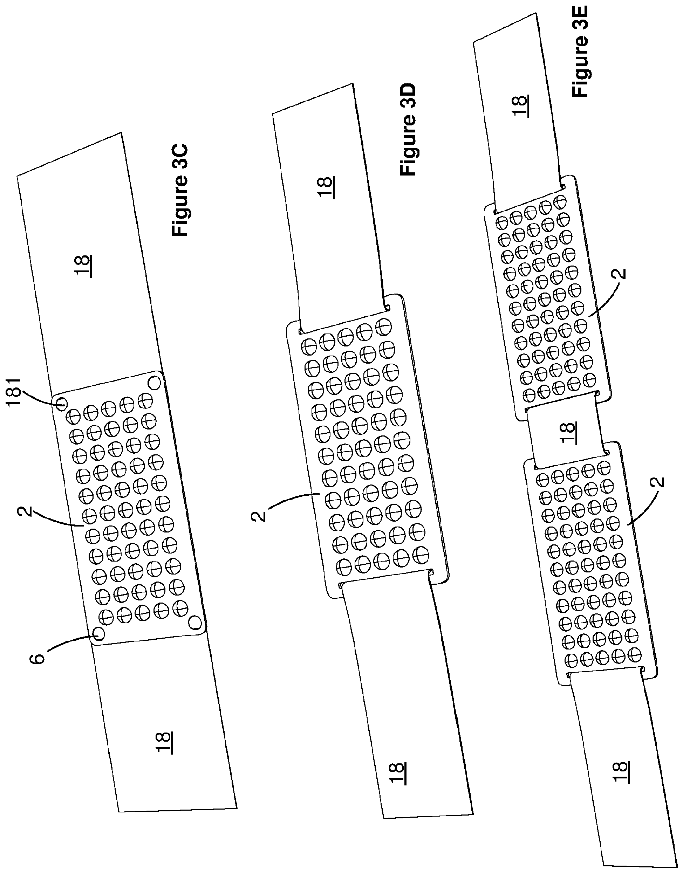

Alternatively or additionally, and as illustrated in Figure 3C, in some examples, the band 2 may be attached to a belt 18, using the fasteners 6 (e.g. headed pins and/or holes). The fasteners 6 may be complementary to fasteners 181 located on the belt 18 (e.g. holes and/or headed pins). In the example of Figure 3C, the adjustment of the loop or the C-shape to the body part of the human or the animal may be provided by the belt 18, and the belt 18 is adjusted to a girth of the body part on which the device 1 is placed. Alternatively or additionally, and as illustrated in Figure 3D, in some examples, the band 2 may attached to a belt 18, using the fasteners 6 comprising a slot. The fasteners such as the slot may be complementary to the width and thickness of the belt 18. In the example of Figure 3D, the belt 18 may pass through the slot and pass at the back of the band. Other embodiments may be envisaged, e.g. two belts may be attached to the band 2, one belt on each side of the band 2 (e.g. on the small sides of the band 2). In the example of Figure 3D, the adjustment of the loop or the C-shape to the body part of the human or the animal may be provided by the belt 18, and the belt is adjusted to a girth of the body part on which the device 1 is placed.

Alternatively or additionally, and as illustrated in Figure 3E, at least two bands 2 may be attached to at least one belt 18, as a non-limiting example.

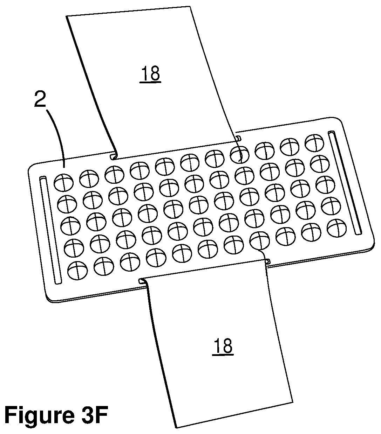

Alternatively or additionally, and as illustrated in Figure 3F, the belt 18 may be attached to the long sides of the band 2.

The belt may be made of a polymer material, or made of fabric as non-limiting examples.

As illustrated in Figure 2, the device 1 comprises a plurality of pads 7, such as eleven rows of five pads as a non-limiting example. As illustrated in Figure 3A, the device 1 may comprise forty-six rows of four pads 7, as a non-limiting example, and as illustrated in Figure 3B, the device 1 may comprise sixty rows of five pads 7, as a non-limiting example, depending on the particular length L and the particular width W, to correspond to the particular body part.

The plurality of pads 7 enable the band 2 to form the loop or the C-shape and enhance the transfer of thermal energy to and/or from the body part of the human or the animal through the receptacle, when the body part of the human or the animal is received in the receiving space 5 defined by the loop or the C-shape.

As illustrated in Figures 2, 3A and 3B, each pad 7 may define a substantially spherical dome, but other shapes may be envisaged as illustrated in Figures 6C, 6D and 6E.

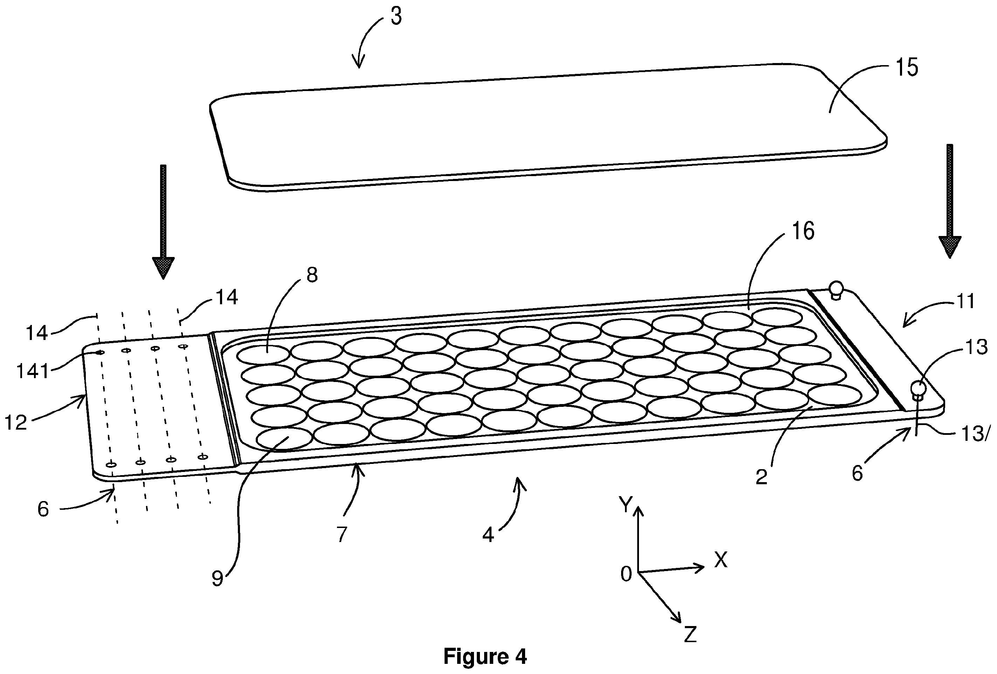

As illustrated in Figure 4, the device 1 may further comprise a lid 15 closing one corresponding aperture 16 of the device 1 (as illustrated by the arrows in Figure 4). In some examples, the aperture 16 is configured to enable filling of the receptacles 8 with the thermal material 9.

As illustrated in Figure 4, the lid 15 is located on the face 3 of the band 2 corresponding to an outer face of the loop or the C-shape.

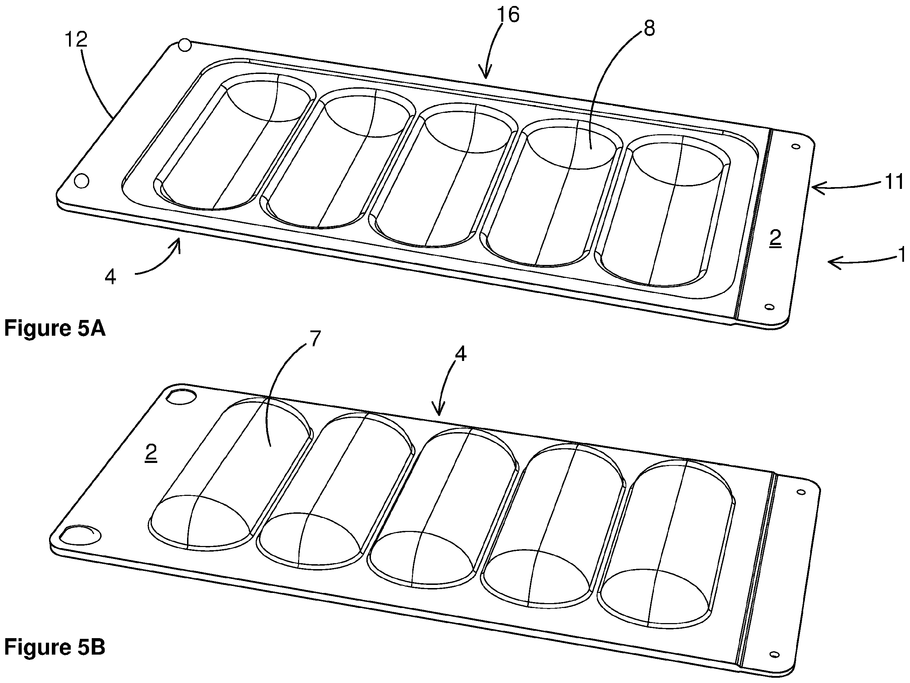

As illustrated in Figures 5A and 5B, the device 1 may comprise a plurality of pads 7, such as five pads as a non-limiting example. In Figures 5A and 5B, each pad 7 pad defines a substantially elongated dome, but other shapes may be envisaged. In Figures 5A and 5B, the elongated dome may be elongated along a long axis perpendicular to the plane (YOX) of Figures 5A and 5B, e.g. parallel to axis (OZ) illustrated in Figures 5A and 5B. The lid closing the aperture 16 of the device 1 is not shown in Figures 5A and 5B.

Alternatively or additionally, in some examples and as illustrated in Figure 6A, the pad 7 may further comprise a bag 17 containing the thermal material 9, the bag 17 being located in the receptacle 8 of the pad 7. The bag 17 may simplify filling of the receptacle 8 with the thermal material 9 and/or may avoid any leakage of the thermal material 9 out of the device 1. The bag 17 may be thin and enable transfer of thermal energy to the body part of the human or the animal and/or transfer of thermal energy from the body part of the human or the animal, through the receptacle 8.

As illustrated in Figure 7, alternatively or additionally, in some embodiments, each pad 7 may be made integral with the polymer material of the band 2, except for an aperture 16 configured to enable filling of the receptacle 8 with the thermal material 9. The device 1 further comprises a lid 15 closing one corresponding aperture 16 of the device 1.

As illustrated in Figure 8, the disclosure also relates to a method 100 for manufacturing a device, e.g. the device 1 according to any aspects of the disclosure, e.g. with reference to Figures 4, 6A and 7.

The method 100 may comprise:

forming, at S1, a band made of a polymer material and configured to form a loop or a C-shape comprising an inner face, the inner face of the loop or the C-shape defining a receiving space for receiving a body part of a human or an animal;
forming, at S2, complementary fasteners, e.g. configured to provide adjustment and/or closing of the loop or the C-shape on the body part of the human or the animal or to enable attachment of the band to one or more belts;
forming, at S3, one or more pads, wherein each pad comprises a receptacle at least substantially made integral with the polymer material of the band; and
filling, at S4, the receptacle with a thermal material configured to enable transfer of thermal energy to and/or from the body part of the human or an animal through the receptacle, when the body part of the human or the animal is received in the receiving space defined by the loop or the C-shape.

Step S4 may involve filling the thermal material through an aperture, e.g. the aperture 16 according to any aspects of the disclosure, and placing a lid, e.g. the lid 15 according to any aspects of the disclosure, on the aperture. The thermal material, e.g. the thermal material 9 according to any aspects of the disclosure, may be placed in a bag, e.g. the bag 17 according to any aspects of the disclosure, before being placed in the receptacle.

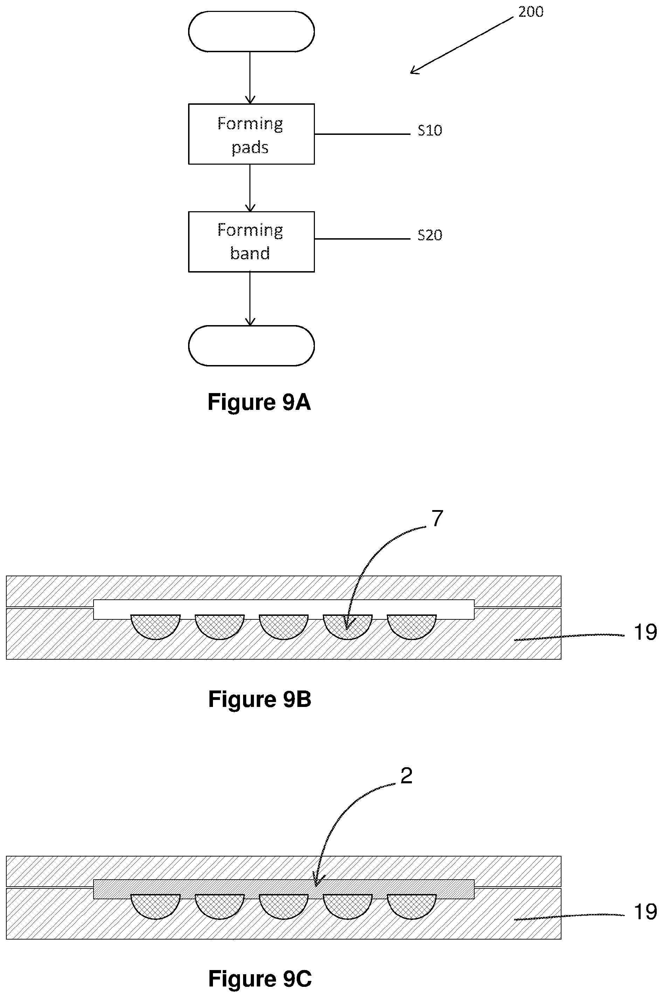

As illustrated in Figures 9A, 9B and 9C, the disclosure also relates to a method 200 for manufacturing a device, e.g. the device 1 according to any aspects of the disclosure, e.g. with reference to Figures 6B, 6C, 6D and 6E.

The method 200 may comprise:

forming, at S10, one or more pads made of a material comprising a mix a polymer material and a thermal material, e.g. in an injection mould 19 as shown in Figure 9B; and
forming, at S20, a band made of a polymer material and configured to form a loop or a C-shape comprising an inner face, the inner face of the loop or the C-shape defining a receiving space for receiving a body part of a human or an animal, such that each pad is least substantially embedded within the polymer material of the band, as shown in Figure 9C.

As illustrated in Figure 9C, the step S20 may be performed in the injection mould 19.

As already stated, the mix of the pad 7 is configured to enable transfer of thermal energy to and/or from the body part of the human or an animal through the pad 7, when the body part of the human or the animal is received in the receiving space defined by the loop or the C-shape.

The method 200 may further comprise forming complementary fasteners, e.g. configured to provide adjustment and/or closing of the loop or the C-shape on the body part of the human or the animal or to enable attachment of the band to one or more belts as disclosed with reference to Figures 3C, 3D, 3E and 3F.

Any one of steps S1, S2, S3 and/or S4, and S10 and/or S20 may be performed via moulding, but other techniques may be envisaged. Any of the steps of the method 100 and/or 200 may involve, as least partly, at least one of e.g. injection, moulding, overmoulding, thermoforming, moulding by compression and/or casting, as non-limiting examples.

The complementary fasteners may be formed integral with the material of the band, or may be attached to the band.

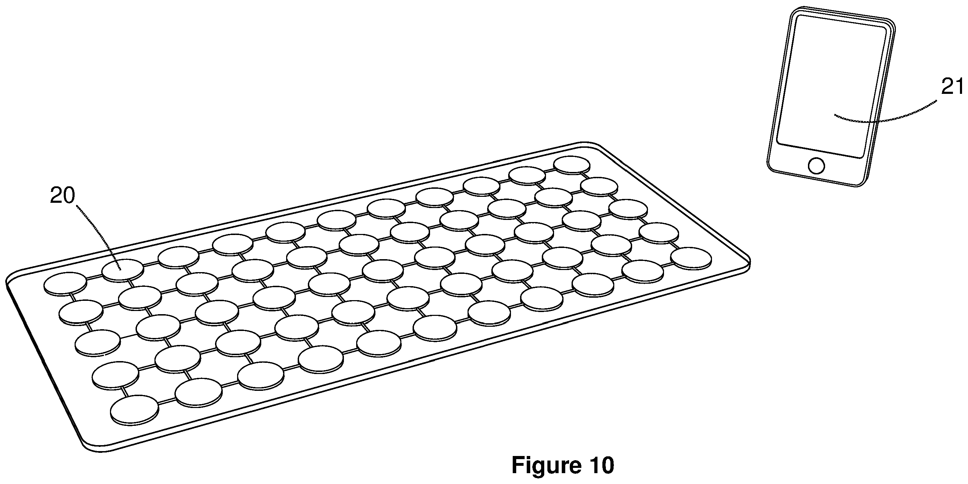

In some embodiments, as illustrated in Figure 10, one or more pads 7 may be replaced by a thermometer 20 configured to measure the temperature of the skin of the human or the animal when the body part of the human or the animal is received in the receiving space defined by the loop or the C-shape. In some examples, the device 1 comprises only thermometers 20. The one or more thermometers may be at least substantially embedded within the polymer material of the band.

In response to the temperature measurement, the one or more thermometers 20 are configured to generate measurement data reflecting the temperature of the skin. In some examples, the thermometers 20 may send the measurement data to a device 21 for processing and/or display, as represented by the arrow in Figure 10. The device 21 may comprise at least one of a distant server, a mobile telephone and/or a dedicated apparatus comprising a processor and/or a display. The dedicated apparatus may be located on the band 2 and may be made integral with or embedded within the material of the band. Alternatively or additionally, the dedicated apparatus may be external to the band, e.g. may be handheld as a non-limiting example.

The sending of the data from the thermometers to the device 21 may be performed over a communication network, and may involve mobile telephone, Wi-Fi and/or Bluetooth protocols, as non-limiting examples.

The thermometers 20 may comprise a processor and a memory and may be powered by a battery, which may be rechargeable.

A rise in the temperature of the skin may indicate inflammation. The device 21 may process the measurement data and display (e.g. on the display of a mobile phone and/or on the display of the dedicated apparatus), based on the processing, information reflecting the location and/or the intensity of the inflammation in the zone covered by the device 1 when the body part of the human or the animal is received in the receiving space defined by the loop or the C-shape.

As illustrated in Figures 11 to 14D, the disclosure also relates to a thermal pad 7. The thermal pad 7 is configured to be in contact with the body part of the human or the animal on either or both of a first face 4 and a second face 3.

The pad 7 comprises a container 22. The container 22 comprises a first part 8 configured to form the first face 4 of the pad 7. The first part 8 is made of a silicone. A second part 15 of the pad 7 is configured to form the second face 3 of the pad 7. The second part 15 may be made of a material comprising a mix of a silicone, and a first thermal material (such as a coolant material and/or a material suitable for heat transfer). The material of the second part 15 enables an enhanced thermal conductivity and/or progressive thermal transfer. The material of the second part 15 enables protection of the skin of the human or the animal against ice burns related to cold and/or thermal burns related to hot.

The first thermal material is configured to enable transfer of thermal energy to and/or from the body part of the human or the animal through the second part 15, when the second face 3 is in contact with the body part of the human or the animal.

The pad 7 also comprises a second thermal material 9 located in the container 22. The second thermal material 9 is configured to enable transfer of thermal energy to and/or from the body part of the human or the animal through the container, when the pad 7 is in contact with the body part of the human or the animal (either on the face 3 and/or the face 4).

In the examples of Figures 11 and 12, the second part 15 may rest on a flange 24 comprising an aperture 25. The flange 24 may be made integral with the material of the first part 8. The aperture 25 may enable filling the container 22 with the second thermal material 9.

The pad 7 of Figures 11 and 12 may be used on its own, e.g. a user may manually hold the pad 7 in contact with the body part of the human or the animal.

Alternatively or additionally, as illustrated in Figures 13A to 13C, the pad 7 may further comprise at least one fastener 6 configured to enable attachment of the pad 7 to at least one fitting 18 configured to provide adjustable attachment of the pad 7 on the body part of the human or the animal. In some examples, the at least one fastener 6 may be located in a band 2 made integral with the first polymer material of the first part 8.

As illustrated in Figure 13A, the at least one fastener 6 may comprise one or more headed pins 131, each forming a button, cooperating with one or more holes 181 of the at least one fitting 18.

It should be understood that other types of fasteners 6 are envisaged.

In some non-limiting examples, the at least one fastener 6 may comprise one or more holes cooperating with one or more headed pins of the at least one fitting, each headed pin forming a button. Alternatively or additionally, the at least one fastener 6 may comprise one or more hooks cooperating with one or more slots or rings of the at least one fitting. Alternatively or additionally, as illustrated in Figure 13B, the at least one fastener 6 may comprise one or more slots 131 (or rings) cooperating with the at least one fitting 18 (such as a belt), although cooperation with one or more hooks of the at least one fitting may also be envisaged. Alternatively or additionally, as illustrated in Figure 13C, the at least one fastener 6 may comprise one or more shoulders 131 cooperating with one or more abutments 181 of the at least one fitting, or any other means such as an abutment or a neck, as non-limiting examples, which enable blocking the pad into and/or attaching the pad to the fitting. As already described in the disclosure, the least one fastener 6 may be configured to enable attachment to one or more belts 18 according to any aspects of the present disclosure.

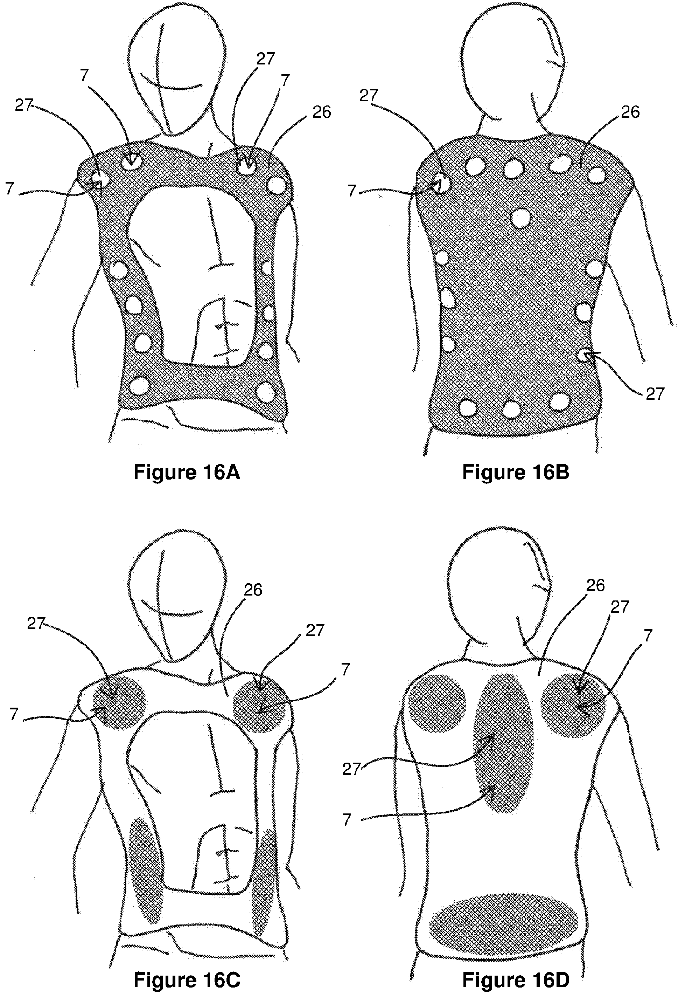

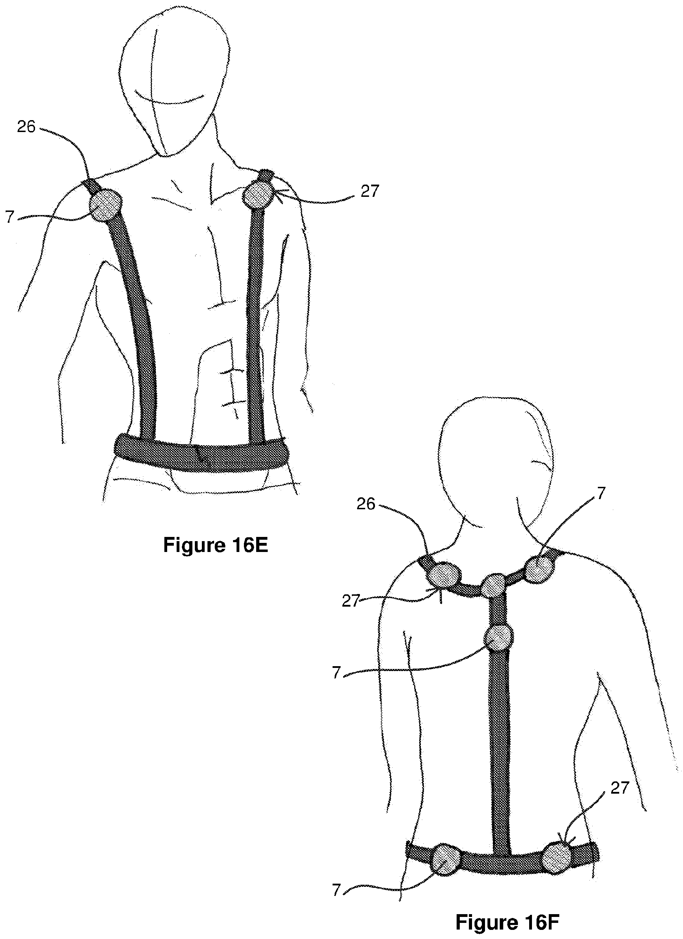

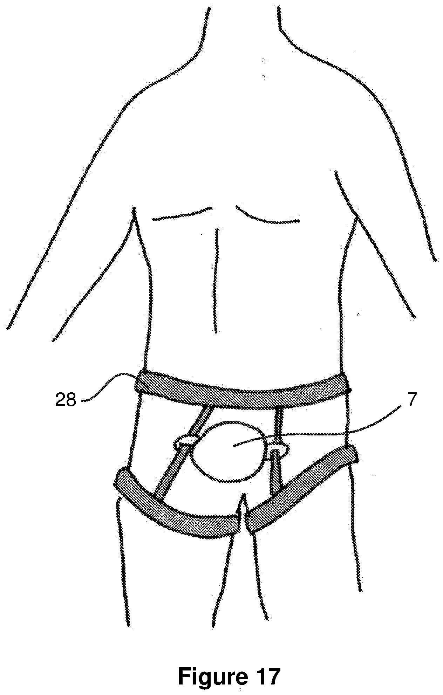

Alternatively or additionally, the fitting may comprise an outfit, such as a jacket (such as a fitting as a jacket 26 as shown in Figures 16A, 16B, 16C, 16D, 16E and 16F), an arm or leg tube, a t-shit, a sleeveless top, shorts, trousers, jumpsuit, a sock, a piece of underwear, a harness (such as a fitting as a harness 28 as shown in Figure 17, for applying the pad 7 to a region corresponding to the pubis - other shapes for the pad 7 than the shape schematically illustrated on Figure 17 are envisaged), tights, a sleeve; and/or an accessory, such as a belt, a Minerva, a scarf and/or a blanket (e.g. for animals - such as a blanket for horses and/or dogs as non-limiting examples), as non-limiting examples. The fitting may comprise any object which can be worn by a user for application of heat and/or cold to the body part.

The fitting may comprise at least one location for receiving the pad. The fitting may comprise a plurality of locations, such that a plurality of pads of any shape or form, may be in contact at several locations with the body of the human or the animal. As shown in Figures 16A, 16B, 16C, 16D, 16E and 16F, the fitting 26 may comprise at least one location 27 for receiving the pad 7. The fitting 26 may comprise a plurality of locations 27, such that a plurality of pads 7 of any shape or form, may be in contact at several locations with the body of the human or the animal (such as front and/or back shoulder areas and/or an upper-back area and/or a lower-back area, as non-limiting examples).

Figures 18A to 18F schematically illustrate several non-limiting examples of shapes and/or locations of the pad 7 on the body part (e.g. the inner shoulder area and/or the arm). Figure 18A shows that the pad 7 may have substantially a semi-sphere shape. Figures 18B and 18F show that the pad 7 may have substantially a revolution cylindrical shape. Figure 18C shows that the pad 7 may have substantially a cross shape. Figure 18D shows that the pad 7 may have substantially a pyramid shape. Figure 18E shows that the pad 7 may have substantially an elongated domed shape.

Additionally or alternatively, as illustrated in Figure 14A, the pad 7 may further comprise at least one suction cup 23 configured to provide attachment of the pad 7 on the body part of the human or the animal. The suction cup 23 may be located in a band 2 made integral with the first polymer material of the first part 8.

Additionally or alternatively, as illustrated in Figures 14B to 14D, the second part 15 may extend beyond the sides of the container 22 to form a flange around the container 22. In Figure 14B, the suction cup 23 of the pad 7 may be located in the flange formed by the second part 15, and made integral with the material of the second part 15.

The container 22 may comprise at least one gusset 33 located at the interface between the first part 8 and the second part 15 to enhance attachment of the pad 7 on the body part of the human or the animal using the suction cup 23. As illustrated in Figure 14B, the container 22 may comprise one gusset 33 located at the interface between the first part 8 and the second part 15. As illustrated in Figure 14C, the container 22 may comprise two successive gussets 33 located at the interface between the first part 8 and the second part 15. As illustrated in Figure 14D, the container 22 may comprise three successive gussets 33 located at the interface between the first part 8 and the second part 15. Other numbers of gussets may be envisaged.

As already described above in the disclosure, the first part 8 may comprises at least one convex curvature facing a direction opposite the container 22 comprising the second thermal material 9 (one curvature is illustrated in Figure 11 and a plurality of curvatures are illustrated in Figure 12). As described above in the disclosure, the at least one convex curvature may be configured to define a substantially spherical dome or a substantially elongated dome, but other shapes may be envisaged, such as a flat face. Alternatively or additionally, the second part 15 may comprise a flat surface facing opposite the container 22 comprising the second thermal material 9, although other shapes for the second part may also be envisaged, such as curved surfaces as non-limiting examples.

The second thermal material 9 has already been described above with reference to e.g. Figure 6A. It should be understood that the second thermal material 9 is configured to be preheated and/or precooled, prior to the pad 7 being placed on the body part of the human or the animal.

As illustrated e.g. in Figures 11 and 12, the first part 8 may have a thickness T1 of polymer material, the thickness T1 being such that:

$$0.4 \text{ mm} \leq T1 \leq 30 \text{ mm}.$$

The second part 15 may have a thickness T2 of material, the thickness T2 being such that:

$$0.4 \text{ mm} \leq T2 \leq 30 \text{ mm}.$$

Alternatively or additionally, as illustrated in Figures 11 and 12 the pad may have a length L, the length L being such that:

$$0.4 \text{ cm} \leq L \leq 150 \text{ cm}.$$

Alternatively or additionally, the pad 7 may have a width W (e.g. along axis (OZ)), the width W being such that:

$$0.4 \text{ cm} \leq W \leq 150 \text{cm}.$$

Alternatively or additionally, the pad 7 may have a height H, the height H being such that:

$$0.4 \text{ cm} \leq H \leq 50 \text{ cm}.$$

The above dimensions are examples only and other dimensions are envisaged.

The disclosure also relates to a thermal device comprising the pad of any aspects of the present disclosure and at least one fitting configured to provide adjustable attachment of the pad on the body part of the human or the animal.

The disclosure also relates to a method 300 of manufacturing a thermal pad, e.g. the pad according to any aspects described with reference to Figures 11 to 14D.

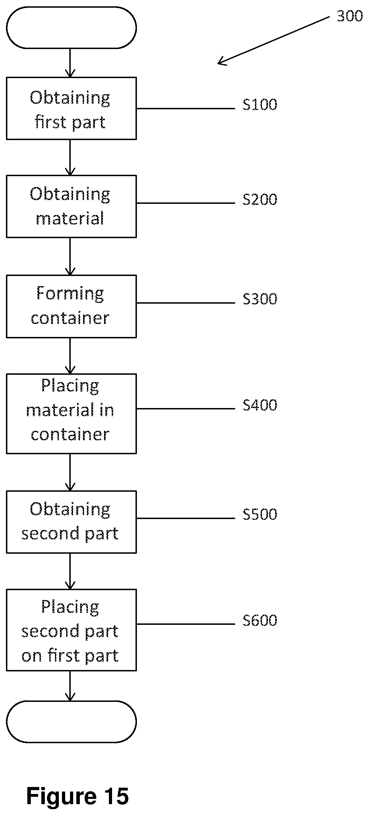

As illustrated in Figure 15, the method 300 comprises:

> obtaining, at S100, a first part configured to form a first face of the pad, the first part being made of a first polymer material;
> obtaining, at S200, a second thermal material, the second thermal material being configured to enable transfer of thermal energy to and/or from a body part of the human or the animal, when the pad is in contact with the body part of the human or the animal;
> forming, at S300, a container using the first part;
> placing, at S400, the second thermal material in the container; and
> obtaining, at S500, a second part configured to form a second face of the pad, the second part being made of a material comprising a mix of:
>
>> a second polymer material, and
>> a first thermal material, the first thermal material being configured to enable transfer of thermal energy to and/or from the body part of the human or the animal, when the second face is in contact with the body part of the human or the animal.

The method 300 may further comprise placing, at S600, the second part on the first part to form the pad.

The method 300 may further comprise forming at least one fastener configured to enable attachment of the pad to at least one fitting configured to provide adjustable attachment of the pad on the body part of the human or the animal. Alternatively or additionally, the method 300 may further comprise forming at least one suction cup configured to provide attachment of the pad on the body part of the human or the animal.

Any one of steps S100, S200, S300, S400, S500 and/or S600 may be performed via moulding, but other techniques may be envisaged. Any of the steps of the method 300 may involve, as least partly, at least one of e.g. injection, moulding, overmoulding, thermoforming, moulding by compression, gluing, extrusion and/or casting, as non-limiting examples.

**Modifications and variations**

The thermal material is under a first physical phase at room temperature (e.g. typically between about 10 degrees Celsius to about 30 degrees Celsius), the first physical phase is a gel, such as an aqueous gel and/or a viscous gel. Alternatively or additionally, the thermal material 9 may be under a second physical phase comprising at least one of: a liquid, a solid and/or a gel, such as an aqueous gel and/or a viscous gel, and/or a powder on its own or diluted, which is different from or the same as the first physical phase, at temperatures other than that of room temperature. Such other temperatures may correspond to temperatures corresponding to a hot oven (e.g. about 100 degrees Celsius to about 200 degrees Celsius, as non-limiting examples), or temperatures corresponding to a refrigerator or a freezer (e.g. about -30 degrees Celsius to about 6 degrees Celsius, as non-limiting examples).

The thermal material 9 may comprise at least one of: water and/or a coolant material, and/or a material suitable for heat transfer.

The polymer material (e.g. for the band and/or the lid and/or the receptacle) comprises at least one of: silicone, polyurethane, thermoplastic elastomer (TPE) and/or elastomer, as non-limiting examples.

The band 2 may comprise holes to allow perspiration when the body part of the human or the animal is received in the receiving space defined by the loop or the C-shape.

In one non-limiting example, the container comprises:

> a first part configured to form a first face of the pad, the first part being made of silicone, and
> a second part configured to form a second face of the pad, the second part being made of a material comprising a mix of:
>
>> a silicone, and
>> a first thermal material, the first thermal material being configured to enable transfer of thermal energy to and/or from the body part of the human or the animal through the second part, when the second face is in contact with the body part of the human or the animal; and
>
> a second thermal material located in the container, the second thermal material being configured to enable transfer of thermal energy to and/or from the body part of the human or the animal through the container, when the pad is in contact with the body part of the human or the animal.

The first polymer material and/or the material comprising a mix of materials may comprise a phosphorescent material.

Other variations and modifications of e.g. the device or pad will be apparent to the skilled in the art in the context of the present disclosure, and various features described above may have advantages with or without other features described above. The above embodiments are to be understood as illustrative examples, and further embodiments are envisaged. Furthermore, equivalents and modifications not described above may also be employed without departing from the scope of the invention, which is defined in the accompanying claims.

**Claims**

1. A mask (1) comprising:

   a thermal pad (7) configured to be in contact with a face of a human or an animal, and
   at least one fitting (18, 26) configured to provide adjustable attachment of the pad (7) on the face of the human or the animal,
   the thermal pad (7) comprising:

      a container (22), the container comprising:

         a first part (8) configured to form a first face (4) of the pad, the first part being made of a silicone, and
         a second part (15) configured to form a second face (3) of the pad, wherein the second face (3) is configured to be in contact with the face of the human or the animal,
         the second part (15) being made of a material comprising a mix of a silicone and a first thermal material, the first thermal material being configured to enable transfer of thermal energy to and/or from the face of the human or the animal through the second part (15), when the second face is in contact with the face of the human or the animal; and

      a second thermal material (9) located in the container (22), the second thermal material being configured to enable transfer of thermal energy to and/or from the face of the human or the animal through the container (22), when the pad is in contact with the face of the human or the animal,

   wherein the second thermal material (9) is configured to be preheated and/or precooled, prior to the mask (1) being placed on the face of the human or the animal, wherein the second thermal material (9) is configured to be a gel at room temperature, from about 10 degrees Celsius to about 30 degrees Celsius, wherein the container (22) further contains one or more further products, wherein the silicone forming the container (22) is configured to enable preservation of the thermal energy which is destined to be transferred to and/or transferred from the face of the human or the animal through the container, when the second face is in contact with the face of the human or the animal, the mask (1) being configured to apply thermal energy to the face of the human or the animal, resulting in vasodilatation, after being preheated and/or to apply cold to the face of the human or the animal, resulting in vasoconstriction, after being precooled, wherein the silicone forming the container (22) is further configured to enable diffusion of the one or more further products located in the container (22) to the face of the human or the animal through the container when the second face is in contact with the face of the human or the animal.

2. The mask of the preceding claim, further comprising at least one fastener (6) configured to enable attachment of the pad to the at least one fitting configured to provide adjustable attachment of the pad on the face of the human or the animal.

3. The mask of the preceding claim, wherein the at least one fastener is located in a band (2) made integral with the first polymer material of the first part.

4. The mask of claim 2 or 3, wherein the at least one fastener is configured to enable attachment to the at least one fitting configured to provide adjustable attachment of the pad on the face of the human or the animal.

5. The mask of any of claims 2 to 4, wherein the at least one fastener comprises at least one of:

   one or more headed pins, each forming a button, cooperating with one or more holes of the at least one fitting;
   one or more holes cooperating with one or more headed pins of the at least one fitting, each headed pin forming a button; and/or
   one or more hooks cooperating with one or more slots or rings of the at least one fitting; and/or one or more slots or rings cooperating with the at least one fitting or one or more hooks of the at least one fitting; and/or one or more shoulders, abutments and/or necks cooperating with the at least one fitting.

6. The mask of any of the preceding claims, wherein the first part comprises at least one convex curvature facing a direction opposite the container comprising the second thermal material, optionally wherein the at least one convex curvature is configured to define a substantially spherical dome or a substantially elongated dome.

7. The mask of any of the preceding claims, wherein the second part comprises a flat surface facing opposite the container comprising the second thermal material.

8. The mask of any of the preceding claims, wherein, the one or more further products comprising at least one of:

   one or more scented materials;
   a cream;
   a serum;
   one or more essential oils;
   vinegar;
   honey;
   starch;
   one or more products for dilatation of the skin or the pores of the skin; and/or one or more products for cleaning and/or sanitizing the skin.

9. The mask of any of the preceding claims, wherein the first polymer material and/or the material comprising a mix of materials comprise a phosphorescent material.

10. The mask of any of the preceding claims, wherein the first part has a thickness T1 of polymer material, the thickness T1 being such that:

$$0.4mm \leq T1 \leq 30mm$$

or

wherein the second part has a thickness T2 of material, the thickness T2 being such that:

$$0.4mm \leq T2 \leq 30mm$$

, or
wherein the pad has a length L, the length L being such that:

$$0.4cm \leq L \leq 150cm$$

; and/or
wherein the pad has a width W, the width W being such that:

$$0.4cm \leq W \leq 150cm$$

; and/or
wherein the pad has a height H, the height H being such that:

$$0.4cm \leq H \leq 50cm.$$

11. The mask (1) of any of the preceding claims, wherein the fitting comprises at least one location for receiving the pad.

12. The mask (1) of any of the preceding claims, configured to be in contact with the face of the human or the animal, in case of a skin trauma and/or skin aggression and/or skin medical operation.

13. The mask (1) of any of the preceding claims, for application before and/or after a surgical operation and/or an aggression on the skin.

**Patentansprüche**

1. Maske (1), umfassend:

   ein Wärmekissen (7), das so gestaltet ist, dass es das Gesicht eines Menschen oder eines Tieres berühren kann, und
   mindestens ein Befestigungselement (18, 26), das so konzipiert ist, dass es eine verstellbare Befestigung des Kissens (7) am Gesicht des Menschen oder des Tieres ermöglicht,
   wobei das Wärmekissen (7) umfasst:

   ein Behältnis (22), wobei das Behältnis umfasst :

   einen ersten Teil (8), der so gestaltet ist, dass er eine erste Seite (4) des Kissens bildet, wobei der erste Teil aus Silikon besteht, und
   einen zweiten Teil (15), der so gestaltet ist, dass er eine zweite Seite (3) des Kissens bildet, wobei die zweite Seite (3) so gestaltet ist, dass sie mit dem Gesicht des Menschen oder Tieres in Kontakt kommt,

   der zweite Teil (15), der aus einer Mischung aus Silikon und einem ersten thermischen Material besteht, wobei das erste thermische Material so konfiguriert ist, dass es die Übertragung von Wärmeenergie zum und/oder vom Gesicht des Menschen oder des Tieres durch den zweiten Teil (15) ermöglicht, wenn die zweite Seite das Gesicht des Menschen oder des Tieres berührt ; und ein zweites thermisches Material (9), das sich in dem Behälter (22) befindet, wobei das zweite thermische Material so konfiguriert ist, dass es die Übertragung von Wärmeenergie zu und/oder von dem Gesicht des Menschen oder des Tieres durch den Behälter (22) ermöglicht, wenn das Kissen mit dem Gesicht des Menschen oder des Tieres in Kontakt ist,
wobei das zweite thermische Material (9) so konfiguriert ist, dass es vorgewärmt und/oder vorgekühlt wird, bevor die Maske (1) auf das Gesicht des Menschen oder des Tieres gelegt wird,
wobei das zweite thermische Material (9) so konfiguriert ist, dass es bei Raumtemperatur, d. h. von etwa 10 °C bis etwa 30 °C, ein Gel ist,
wobei der Behälter (22) zusätzlich ein oder mehrere andere Produkte enthält,
wobei das den Behälter (22) bildende Silikon so konfiguriert ist, dass es die Speicherung der Wärmeenergie ermöglicht, die durch den Behälter auf das Gesicht des Menschen oder des Tieres übertragen und/oder von diesem übertragen werden soll, wenn die zweite Seite mit dem Gesicht des Menschen oder des Tieres in Kontakt ist,
wobei die Maske (1) so konfiguriert ist, dass sie nach dem Vorwärmen Wärmeenergie auf das Gesicht des Menschen oder des Tieres überträgt, was zu einer Vasodilatation führt, und/oder Kälte auf das Gesicht des Menschen oder des Tieres überträgt, was zu einer Vasokonstriktion führt, nachdem es vorgekühlt wurde,
wobei das den Behälter (22) bildende Silikon außerdem so konfiguriert ist, dass es die Diffusion eines oder mehrerer anderer Produkte, die sich in dem Behälter (22) befinden, durch den Behälter auf das Gesicht des Menschen oder des Tieres ermöglicht, wenn die zweite Seite mit dem Gesicht des Menschen oder des Tieres in Kontakt ist.

2. Die oben beschriebene Maske, die außerdem mindestens ein Befestigungselement (6) umfasst, das so konfiguriert ist, dass es die Befestigung des Kissens an mindestens einem Halteelement ermöglicht, das so konfiguriert ist, dass es eine verstellbare Befestigung des Kissens am Gesicht des Menschen oder des Tieres ermöglicht.

3. Die oben beschriebene Maske wobei sich mindestens ein Befestigungselement in einem Streifen (2) befindet, der in das erste Polymermaterial des ersten Teils integriert ist.

4. Die Maske nach claim 2 oder 3, wobei mindestens ein Befestigungselement so konfiguriert ist, dass es die Befestigung an mindestens einem Halteelement ermöglicht, das so konfiguriert ist, dass es eine einstellbare Fixierung des Kissens auf dem Gesicht des Menschen oder des Tieres gewährleistet.

5. Die Maske nach einem der Ansprüche 2 bis 4, wobei mindestens ein Befestigungselement mindestens eines der folgenden Elemente umfasst:

   einen oder mehrere Kopfstifte, die jeweils einen Knopf bilden und so konfiguriert sind, dass sie in ein oder mehrere Löcher in dem/den Halteelement/en passen;
   ein oder mehrere Löcher, die mit einem oder mehreren Kopfstiften in dem/den Halteelement/en zusammenwirken, wobei jeder Kopfstift einen Knopf bildet; und/oder
   einen oder mehrere Haken, die so konfiguriert sind, dass sie in einen oder mehrere Schlitze oder Ringe des oder der Halteelemente passen; und/oder
   einen oder mehrere Schlitze oder Ringe, die mit einem oder mehreren Halteelementen oder einem oder mehreren Haken des oder der Halteelemente zusammenwirken; und/oder
   einen oder mehrere Flansche, Anschläge und/oder Verengungen, die mit mindestens einem Halteelement zusammenwirken.

6. Maske nach einem der vorhergehenden Ansprüche, wobei der erste Teil mindestens eine konvexe Krümmung aufweist, die von dem Behälter, der das zweite thermische Material enthält, weg weist, wobei die mindestens eine konvexe Krümmung optional so konfiguriert ist, dass sie eine im Wesentlichen kugelförmige Kuppel oder eine im Wesentlichen längliche Kuppel definiert.

7. Maske nach einem der vorhergehenden Ansprüche, wobei der zweite Teil eine ebene Oberfläche umfasst, die von dem Behälter weg gerichtet ist, der das zweite thermische Material umfasst.

8. Die Maske nach einem der vorhergehenden Ansprüche, wobei das oder die anderen Produkte mindestens eines der folgenden umfassen:

   einen oder mehrere Duftstoffe;
   eine Creme;

ein Serum;
ein oder mehrere ätherische Öle;
Essig;
Honig;
Stärke;
ein oder mehrere Produkte zur Erweiterung der Haut oder der Hautporen;
und/oder
ein oder mehrere Produkte zur Reinigung und/oder Klärung der Haut.

9. Maske nach einem der vorhergehenden Ansprüche, wobei das erste Polymermaterial und/oder das Material, das eine Mischung von Materialien umfasst, ein phosphoreszierendes Material umfasst.

10. Die Maske nach einem der vorhergehenden Ansprüche, wobei der erste Teil eine Dicke T1 des Polymermaterials aufweist, wobei die Dicke T1 so ist, dass:

$$0,4 \text{ mm} \leq T1 \leq 30 \text{ mm},$$

oder

wobei der zweite Teil eine Dicke T2 des Materials aufweist, wobei die Dicke T2 so ist, dass:

$$0.\ 4\text{mm} \leq T2 \leq 30\text{mm},$$

, oder
wobei das Kissen eine Länge L aufweist, wobei die Länge L so ist, dass:

$$0.4\text{cm} \leq L \leq 150\text{cm}$$

; und/oder
wobei das Kissen eine Breite W aufweist, wobei die Breite W so ist, dass:

$$0.4\text{cm} \leq W \leq 150\text{cm}$$

; und/oder
wobei das Kissen eine Höhe H aufweist, wobei die Höhe H so ist, dass:

$$0.4\text{cm} \leq H \leq 50\text{cm}.$$

11. Maske (1) nach einem der vorhergehenden Ansprüche, wobei die Haltevorrichtung mindestens eine Stelle zur Aufnahme des Kissens umfasst.

12. Die Maske (1) nach einem der vorhergehenden Ansprüche, die so konfiguriert ist, dass sie im Falle eines Hauttraumas und/oder eines Hautangriffs und/oder eines medizinischen Eingriffs an der Haut mit dem Gesicht des Menschen oder des Tieres in Kontakt kommt.

13. Maske (1) nach einem der vorhergehenden Ansprüche, zur Anwendung vor und/oder nach einem chirurgischen Eingriff und/oder einer Aggression auf der Haut.

**Revendications**

1. Masque (1) comprenant :

un tampon thermique (7) configuré pour être en contact avec le visage d'un humain ou d'un animal, et
au moins un élément de fixation (18, 26) conçu pour permettre la fixation ajustable du tampon (7) sur le visage de l'humain ou de l'animal,
le tampon thermique (7) comprenant :

un contenant (22), le contenant comprenant :

une première partie (8) configurée pour former une première face (4) du tampon, la première partie étant faite de silicone, et
une seconde partie (15) configurée pour former une seconde face (3) du tampon, la seconde face (3) étant configurée pour être en contact avec le visage de l'humain ou de l'animal,
la seconde partie (15) étant composée d'un matériau comprenant un mélange de silicone et d'un premier matériau thermique, le premier matériau thermique étant configurée pour permettre le transfert d'énergie thermique vers et/ou depuis le visage de l'humain ou de l'animal à travers la seconde partie (15), lorsque la seconde face est en contact avec le visage de l'humain ou de l'animal ; et

un second matériau thermique (9) situé dans le contenant (22), le second matériau thermique étant configuré pour permettre le transfert d'énergie thermique vers et/ou depuis le visage de l'humain ou de l'animal à travers le contenant, (22), lorsque le tampon est en contact avec le visage de l'humain ou de l'animal,
dans lequel le second matériau thermique (9) est configuré pour être préchauffé et/ou pré-refroidi, avant que le masque (1) ne soit placé sur le visage d'un humain ou d'un animal,
dans le lequel le second matériau thermique (9) est configuré pour être un gel à température ambiante, compris entre environ 10 °C et environ 30 °C, dans lequel le contenant (22) contient en outre un ou plusieurs autres produits, dans lequel le silicone formant le contenant (22) est configuré pour permettre la conservation de l'énergie thermique qui doit être transférée vers et/ou transférée du visage de l'homme ou de l'animal à travers le contenant, lorsque la seconde face est en contact avec le visage de l'homme ou de l'animal, le masque (1) étant configuré pour appliquer de l'énergie thermique au visage de l'homme ou de l'animal, entraînant une vasodilatation, après avoir été préchauffé et/ou appliquer du froid sur le visage de l'homme ou de l'animal, entraînant une vasoconstriction, après avoir été prérefroidi, dans lequel le silicone formant le contenant (22) est en outre configuré pour permettre la diffusion d'un ou de plusieurs autres produits situés dans le recipient (22) sur le visage de l'homme ou de l'animal à travers le contenant lorsque la seconde face est en contact avec le visage de l'homme ou de l'animal.

2. Le masque selon la revendication précédente, comprenant en outre au moins un élément de fixation (6) configuré pour permettre la fixation du tampon sur au moins un élément de maintien configuré pour permettre une fixation réglable du tampon sur le visage de l'être humain ou de l'animal.

3. Le masque selon la revendication précédente, dans lequel au moins un élément de fixation est situé dans une bande (2) intégrée au premier matériau polymère de la première partie.

4. Le masque selon la revendication 2 ou 3, dans lequel au moins un élément de fixation est configure pour permettre la fixation à au moins un élément de maintien configuré pour assurer une fixation ajustable du tampon sur le visage de l'humain ou de l'animal.

5. Le masque selon l'une quelconque des revendications 2 à 4, dans lequel au moins un élément de fixation comprend au moins l'un des éléments suivants :

   un ou plusieurs picots à tête, formant chacun un bouton, configurés pour s'adapter dans un ou plusieurs trous du ou des éléments de maintien; un ou plusieurs trous cooperant avec un ou plusieurs picots à tête du ou des éléments de maintien, chaque picot à tête formant un bouton;

et/ou
   un ou plusieurs crochets configurés pour s'adapter avec une ou plusieurs fentes ou oeillets du ou des éléments de maintien; et/ou
   une ou plusieurs fentes ou oeillets cooperant avec un ou des éléments de maintien ou un ou plusieurs crochets du ou des éléments de maintien; et/ou
   un ou plusieurs rebords, butées et/ou rétrécissement coopérant avec au moins un élément de maintien.

6. Le masque de l'une quelconque des revendications précédentes, dans lequel la première partie comprend au moins une courbure convexe orientée vers une direction opposée au contenant comprenant le second matériau thermique, dans lequel au moins une courbure convexe est éventuellement configurée pour définir un dôme sensiblement sphérique ou un dôme sensiblement allongé.

7. Le masque de l'une quelconque des revendications précédentes, dans lequel la seconde partie comprend une surface plane orientée vers l'opposé du contenant comprenant le second matériau thermique.

8. Le masque selon l'une quelconque des revendications précédentes, dans lequel le ou les autres produits comprennent au moins l'un des éléments suivants :

   une ou plusieurs substances parfumées ;
   une crème ;
   un sérum ;
   une ou plusieurs huiles essentielles ;
   du vinaigre ;
   du miel ;
   de l'amidon ;
   un ou plusieurs produits destinés à la dilatation de la peau ou des pores de la peau ; et/ou
   un ou plusieurs produits destinés au nettoyage et/ou à la purification de la peau.

9. Le masque selon l'une quelconque des revendications précédentes, dans lequel le premier matériau polymère et/ou le matériau comprenant un mélange de matériaux comprend un matériau phosphorescent.

10. Le masque selon l'une quelconque des revendications précédentes, dans lequel la première partie présente une épaisseur T1 de matériau polymère, ladite épaisseur T1 étant telle que :

$$0{,}4 \text{ mm} \leq T1 \leq 30 \text{ mm}$$

, ou

dans lequel la seconde partie présente une épaisseur T2 de matériau, ladite épaisseur T2 étant telle que :

$$0.4mm \leq T2 \leq 30mm,$$

, ou

dans lequel le tampon présente une longueur L, ladite longueur L étant telle que :

$$0.4cm \leq L \leq 150cm$$

; et/ou

dans lequel le tampon présente une largeur W, ladite largeur W étant telle que :

$$0.4cm \leq W \leq 150cm;$$

; et/ou

dans lequel le tampon présente une hauteur H, ladite hauteur H étant telle que :

$$0.4cm \leq H \leq 50cm.$$

11. Le masque (1) selon l'une quelconque des revendications précédentes, dans lequel le dispositif de maintien comprend au moins un emplacement destiné à recevoir le tampon.

12. Le masque (1) selon l'une quelconque des revendications précédentes, configuré pour être en contact avec le visage de l'homme ou de l'animal, en cas de traumatisme cutané et/ou d'agression cutanée et/ou d'opération médicale cutanée.

13. Le masque (1) selon l'une quelconque des revendications précédentes, pour application avant et/ou après une opération chirurgicale et/ou une agression sur la peau.

**Figure 1**

**Figure 2**

**Figure 3A**

**Figure 3B**

17

Figure 3C

Figure 3D

Figure 3E

Figure 3F

**Figure 4**

EP 3 897 485 B1

EP 3 897 485 B1

Figure 5A

Figure 5B

21

**Figure 6A**

**Figure 6B**

**Figure 6C**

**Figure 6D**

**Figure 6E**

**Figure 7**

**Figure 8**

200

Forming
pads — S10

Forming
band — S20

**Figure 9A**

7

19

**Figure 9B**

2

19

**Figure 9C**

Figure 10

Figure 11

Figure 12

Figure 13 A

Figure 13 B

Figure 13 C

**Figure 14A**

**Figure 14B**

**Figure 14C**

**Figure 14D**

300

S100 — Obtaining first part

S200 — Obtaining material

S300 — Forming container

S400 — Placing material in container

S500 — Obtaining second part

S600 — Placing second part on first part

**Figure 15**

**Figure 16A**

**Figure 16B**

**Figure 16C**

**Figure 16D**

**Figure 16E**

**Figure 16F**

**Figure 17**

**Figure 18A**

**Figure 18B**

**Figure 18C**

**Figure 18D**

**Figure 18E**

**Figure 18F**

**EP 3 897 485 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1782778 A
- US 2018042762 A
- US 2017332796 A
- EP 2881092 A
- WO 2014066842 A